(19) Europäisches Patentamt
European Patent Office
Office européen des brevets

(11) Publication number: 0 066 124
B1

(12) **EUROPEAN PATENT SPECIFICATION**

(45) Date of publication of patent specification: **19.09.84**

(21) Application number: **82104051.6**

(22) Date of filing: **10.05.82**

(51) Int. Cl.$^3$: **C 07 D 495/14,**
C 07 D 495/22, A 61 K 31/495
// C07D495/04 ,(C07D495/14, 333/00, 237/00, 221/00)

(54) **Pyridothienopyridazine compounds.**

(30) Priority: **18.05.81 US 264756**

(43) Date of publication of application:
**08.12.82 Bulletin 82/49**

(45) Publication of the grant of the patent:
**19.09.84 Bulletin 84/38**

(84) Designated Contracting States:
**BE CH DE FR GB IT LI NL SE**

(56) References cited:
**No relevant documents have been disclosed.**

(73) Proprietor: **USV PHARMACEUTICAL CORPORATION**
**1 Scarsdale Road**
**Tuckahoe New York (US)**

(72) Inventor: **Youssefyeh, Raymons D.**
**435 Martling Avenue**
**Tarrytown New York (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 066 124 B1

**Description**

The invention relates to new chemical compounds possessing valuable pharmaceutical activity. It particularly relates to pyridothienopyridazines possessing useful anti-allergy activity and having the structure

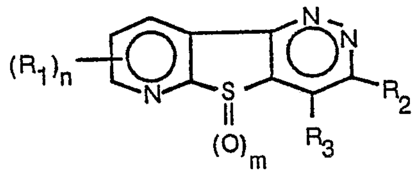

I

wherein

$R_1$ is alkyl; alkenyl, alkynyl, halogen, nitro, cyano, carboxyl, carboxyalkyl, carbalkoxy, alkanoyl, trihalomethyl, aryl, aralkyl, mercapto, mercaptoalkyl, alkylmercapto, hydroxy, hydroxyalkyl, alkoxy, acyloxy, amino, aminoalkyl, alkylamino, sulfinyl, sulfonyl or methylenedioxy,

m is an integer from 0 to 2 inclusive,

n is an integer from 0 to 3 inclusive, and

$R_2$ and $R_3$ are independently hydrogen, alkyl, alkenyl, alkynyl, hydroxy, hydroxyalkyl, alkoxy, acyloxy, halogen, amino, aminoalkyl, alkylamino, alkanoylamino, mercapto, mercaptoalkyl, alkylmercapto, carboxy, carbalkoxy, alkylsulfinyl, alkylsulfonyl, arylsulfinyl, or arylsulfonyl.

The alkyl groups in alkyl per se and in hydroxyalkyl alkoxy, mercaptoalkyl, alkylmercapto, aminoalkyl, alkylamino, alkanoyl, carbalkoxy, aralkyl, alkylsulfinyl and alkylsulfonyl are either straight-chain or branched lower alkyl groups containing from 1 to 6 carbon atoms.

The acyl group of the aforesaid acyloxy groups is any organic acid radical from which the radical OH has been removed from the acid group. Thus, the corresponding radicals of the following acids are preferred: acetic, benzoic, isobutyric, pentanoic, cyclohexanecarboxylic, toluic, toluenesulfonic, napthalenesulfonic, phenylacetic, methanesulfonic, and similar acids. Hydrocarbylcarboxylic and sulfonic acids in which the hydrocarbyl group is alkyl, cycloalkyl, aryl and aralkyl, preferably containing up to a total of 10 carbon atoms are preferred.

The alkenyl and alkynyl groups may be straight-chain or branched and contain from 2 to 6 carbon atoms.

The aryl groups contain froma 6 to 10 carbon atoms and include phenyl, $\alpha$-naphthyl, and $\beta$-naphthyl. These groups may carry substituents such as alkyl, alkenyl, alkynyl, hydroxy, alkoxy, mercaptoalkyl, alkylmercapto, amino, alkylamino, cyano, carboxy, carbalkoxy, sulfinyl, sulfonyl, trifluoromethyl, methylenedioxy, halogen, and nitro.

The halogen substituents are preferably chlorine, bromine and trifluoromethyl.

The compounds of the present invention are capable of forming salts with acids and when acid groups such as carboxyl groups are present, salts with bases. Preferred are salts formed with pharmaceutically-acceptable acids and bases. Suitable acid addition salts include salts of inorganic acids such as hydrochloric, sulfuric and phosphoric and organic acids such as acetic, lactic, benzoic, nicotinic, malic, succinic, tartaric, citric, mandelic and the like.

Suitable basic salts include salts of alkali and alkaline earth metals, iron, and amines.

The preferred compounds of the present invention are those wherein n is 1, $R_1$ is lower alkyl, $R_2$ is carboxy or carbalkoxy and $R_3$ is hydroxy or alkoxy.

The compounds of the present invention may be prepared by the following reaction:

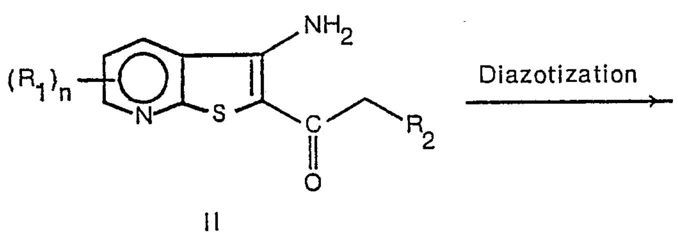


I

II

Especially preferred are compounds of formula II in which $R_2$ is carbalkoxy. The $R_2$ carbalkoxy group can be readily saponified to a free carboxylic acid and, if desired, the carboxyl group removed, e.g., by heating or by replacement reactions.

Compounds of structure II can be prepared by reaction of a compound of the formula:

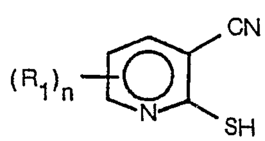

III

with a compound of the formula:

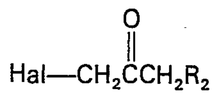

IV

especially where $R_2$ is carbalkoxy, with the formation of hydrogen halide as a by-product.

Compounds of structure I can also be prepared by ring closure of a compound of the formula:

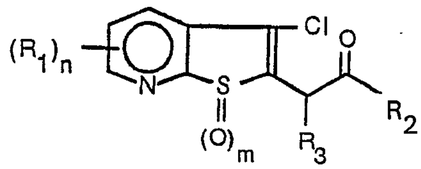

V

with hydrazine; or by diazotization and ring closure of a compound of the formula:

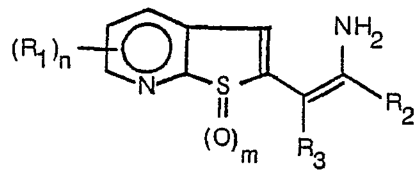

VI

or by ring-closure of compounds of the formulae:

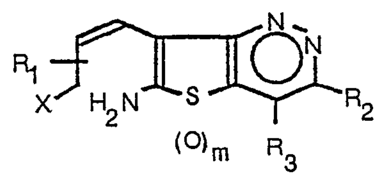

VII

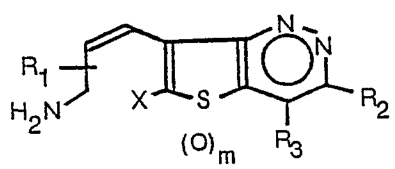

VIII

by elimination of HX and aromatization of the dihydropyridine ring.

The compounds of formula I wherein m = 1 or 2, i.e., the cyclic sulfoxides and cyclic sulfones corresponding to the thieno ring, can also be prepared by reaction of compounds of structure I in which m = 0 with peroxides or peracids such as hydrogen peroxide, isobutylperoxide, benzoylperoxide, peracetic acid and perbenzoic acid which are exemplary but not exhaustic of the reagents to be used for the oxidation. However, the reaction conditions may lead to formation of N-oxides, especially with pyridazine ring nitrogen atoms.

When the compounds produced include a 4-hydroxy group (as obtained, for example, from compounds of structure II herein) the 4—OH group can be modified as by etherification with hydrocarbyl groups by reaction with hydrocarbyl halides by removal of hydrogen halide or equivalent derivatives such as dihydrocarbyl sulfates and the like. Alternatively, the 4—OH group can be acylated with organic acids to form corresponding esters such as acetates, benzoates, sulfonates, phosphates, malonates, citrates and the like. If desired, the 4—OH can be replaced by a variety of substituents, e.g., halide, or simply removed as by catalytic hydrogenolysis or other known reactions such as chemical reduction.

Various substituents can be introduced into the products produced by the hereindescribed synthetic procedures by the usual substitution reactions such as alkylation, nitration, acylation, and the like or by conversion of existing substituents such as nitro to amino by reduction and amino to halo by diazotization and replacement of the diazo group with halo and equivalent groups.

The aforesaid reactions can be conveniently carried out in organic solvents at temperatures ranging from 0° to the reflux temperature of the reaction mixture. The reaction periods are determined by the temperature selected for the reaction and the nature of the reactants. Progress of the reactions can be determined by removal of aliquots from the reaction mixture and analysis for either the reactants, the products, or both to determine the relative concentrations thereof. As is usual in organic reactions, even after substantially complete reaction mixtures are digested by heating for extended periods usually at the reflux temperature of the reaction mixture.

Commonly, the solvent selected for the foregoing reactions is water-miscible in which case solvents such as dioxane, ethanol, methanol, acetone, dimethylformamide, diethylacetamide, and tetrahydrofuran are used. Water immiscible solvents can also be used.

In those reactions where hydrogen halide is formed, the reactions are preferably accomplished in the presence of a hydrogen halide acceptor such as inorganic bases, e.g., sodium hydroxide, sodium carbonate and lime, or organic bases such as amines, e.g., trimethylamine, aniline, pyridine and the like.

The diazotization reactions called for in the foregoing are accomplished by known procedures employing, for example, sodium nitrite or equivalent nitrite such as isoamynitrite in the presence of an acid such as acetic acid or a mineral acid such as hydrochloric or sulfuric acid. These reactions are usually conducted at about room temperature and even lower, e.g., to about 0°C.

Aromatization can be accomplished by known methods by reacting with reagents to remove hydrogen from the substrate compound. Thus, the substrate can be heated with sulfur or other such reagent, e.g., 2,3-dichloro-5,6-dicyano-1,4-benzoquinone, to prepare the fully aromatized product, particularly, the aromatized pyrido ring.

It is to be understood that certain compounds of this invention may have asymmetric carbon atoms and thus may exist in racemic or optically active forms. These different forms are considered to be within the scope of the present invention.

The invention is further illustrated by the following examples.

Example 1

*3-Carbomethoxy-4-Hydroxy-7-Methyl-Pyrido-[3′,2′—4,5]-Thieno[3,2—C]Pyridazine*

To a solution of 6 g (0.22 mol) of 3-amino-2-carbomethoxyacetyl-6-methyl-thieno[2,3-b]pyridine in 1.8 ml acetic acid and 180 ml dioxane was slowly added 4.8 g (4.2 ml, 0.041 mol) of isoamylnitrite and stirring continued for 24 hours. It was then poured on ice-water, filtered and washed with water. The crude product was crystallized from DMSO/$H_2O$ to give 4.0 g (64%) of 3-carbomethoxy-4-hydroxy-7-methylpyrido[3′,2′:4,5]-thieno-[3,2-c]pyridazine; m.p., 265—268°C.

Example 2

*3-Carboxy-4-Hydroxy-7-Methyl-Pyrido[3′,2′:4,5]Thieno-[3,2—C]Pyridazine*

A mixture of 4 g (0.015 mol) of 3-carbomethoxy-4-hydroxy-7-methyl-pyrido[3′,2′:4,5]thieno[3,2—C]pyridazine in 60 ml of 3% NaOH solution was stirred at room temperature for 2 hours. It was then diluted with water, treated with charcoal and filtered. It was acidified with acetic acid and filtered. Crystallization of the crude product from 3% NaOH/HOAC gave 2 g (53%) of 3-carboxy-4-hydroxy-7-methylpyrido[3′,2′:4,5]thieno[3,2—C]pyridazine, m.p. > 300°C.

Example 3

*4-Hydroxy-7-Methyl-Pyrido-[3′,2′:4,5]-Thieno-[3,2—C]-Pyridazine*

A mixture of 2.4 g (0.009 mol) of 3-carboxy-4-hydroxy 7-methylpyrido[3′,2′:4,5]-thieno[3,2—C]pyridazine in 70 ml of Dowtherm-A® was stirred at 225°C. for 30 minutes. It was cooled, diluted with hexane and filtered. The crude product was crystallized by 5% NaOH/HOAC giving 1.3 g (65%) of 4-hydroxy-7-methyl-pyrido[3′,2′:4,5]thieno[3,2—C]pyridazine, m.p. >300°C.

Example 4

*4-Hydroxy-7-Methyl-Pyrido[3′,2′:4,5]Thieno-[3,2—C]Pyridazine-3-Carboxylate Sodium Salt*

To a solution of 370 mg (0.016 mol) of sodium in 250 ml ethanol was added 4.2 g (0.016 mol) of 3-carbethoxy-4-hydroxy-7-methyl-pyrido[3′,2′:4,5]thieno[3,2—C]pyridazine and stirring was continued for two hours. Solvent was then removed, the residue was diluted with 30 ml water and the product was filtered giving 3.3 g of the sodium salt of 3-carboxy-4-hydroxy-7-methyl-pyrido[3′,2′:4,5]thieno[3,2—C]pyridazine; m.p. > 300°C.

Example 5

*3-Carbethoxy-4-Ethoxy-7-Methyl-Pyrido[3′,2′:4,5]Thieno[3,2—C]-Pyridazine*

To a mixture fo 3.3 g (0.013 mol) of 3-carbomethoxy-4-hydroxy-7-methyl-pyrido[3′,2′:4,5]thieno[3,2—C]-pyridazine, 3.8 g potassium bicarbonate, in 90 ml of anhydrous DMF was slowly added 9 ml of ethyl bromide and stirring was continued at 60—70°C. for 3 hours. The solvent was then removed, the residue was diluted with water and filtered to give 3.4 g of crude product. Crystallization from EtoAc-hexane gave 1.8 g (46%) of pure 3-carbethoxy-4-ethoxy-7-methyl-pyrido[3′,2′:4,5]thieno-[3,2—C]pyridazine; m.p. 158—160°C.

Example 6

Step A

*3-Amino-2-[2-(4-chlorophenylsulfonyl)-acetyl]-6-methyl-thieno[2,3-b]-pyridine*

To a mixture of 5.4 g (0.1 mol) of sodium methoxide and 7.5 g (0.05 mol) of 2-mercapto-3-cyano-6-methyl-pyridine in 100 ml methanol was slowly added 13.3 g (0.05 mol) of 3-chloro-1-(4-chloro-phenylsulfonyl)-2-propanone. The reaction mixture was refluxed for 7 hours. It was then cooled, diluted with water and filtered to give 15.5 g of crude product which was crystallized from acetonitrile to give 12.8 g of pure product, m.p. 265—7°C.

Step B

*3-(4-Chlorophenylsulfonyl)-4-hydroxy-7-methyl-pyrido[3',2':4,5]thieno[3,2—C]pyridazine*

To a mixture of 4 g of 3-amino-2-[2-(4-chlorophenylsulfonyl)-acetyl]-6-methyl-thieno-[2,3-b]pyridine in 100 ml of 5% hydrochloric acid was slowly added 2.1 g sodium nitrite in 20 ml water and stirring was continued for 24 hours. To this mixture was then added a solution of 1.8 g sodium nitrite in 30 ml of 5% hydrochloric acid and stirring was continued for another 24 hours. The reaction was diluted with water and filtered to give crude product which was crystallized from DMSO, m.p. > 300°C.

Following the procedures of the above examples, the following additional compounds can be prepared:

3-Carboxy-4-hydroxy-pyrido[3',2':4,5]thieno[3,2-c]pyridazine
3-Cyano-4-hydroxy-7-phenyl-pyrido[3',2':4,5]thieno[3,2-c]pyridazine
3-Carboxy-4-chloro-7,9-dimethyl-pyrido[3',2':4,5]thieno[3,2-c]pyridazine
3-Carbamido-4-chloro-7,9-diphenyl-pyrido[3',2':4,5]thieno[3,2-c]pyridazine
3-Carboxy-4-amino-7-methyl-9-phenyl-pyrido[3',2':4,5]thieno[3,2-c]pyridazine
3-Carboxy-4-hydroxy-7-methyl-9-chloro-pyrido[3',2':4,5]thieno[3,2-c]pyridazine
3-Carbamido-4-hydroxy-7,8-dimethyl-pyrido[3',2':4,5]thieno[3,2-c]pyridazine
3-Carboxy-4-hydroxy-7,8-diphenyl-pyrido[3',2':4,5]thieno[3,2-c]pyridazine
3-Acetyl-4-ethoxy-7-methyl-8-phenyl-pyrido[3',2':4,5]thieno[3,2-c]pyridazine
3-Carboxy-4-hydroxy-7-methyl-8-cyano-pyrido[3',2':4,5]thieno[3,2-c]pyridazine
3-Sulfonic acid-4-hydroxy-7-methyl-8-trifluoromethyl-pyrido[3',2':4,5]thieno[3,2-c]pyridazine
3-Sulfonic acid-4-hydroxy-7-ethyl-8-nitro-pyrido[3',2':4,5]thieno[3,2-c]pyridazine
3,8-Dicarboxy-2-hydroxy-7-phenyl-pyrido[3',2':4,5]thieno[3,2-c]pyridazine

The compounds of the present invention show strong activity as inhibitors of wheal formation in the passive cutaneous anaphylaxis (PCA) screen and as inhibitors of histamine release from passively sensitized rat mast cells (RMC), making them useful in the treatment of allergy. The compounds exhibit $ED_{50}$ values of from 1.0 to 50.0 mg/kg on oral administration in the PCA screen and $I_{50}$ values of from 1 to 100 $\mu$m in the RMC screen. The compounds may be administered orally or parenterally in the treatment of allergies and related conditions, and it will be within the skill of the practitioner to determine the exact amount to be administered and the mode of administration.

## Claims

1. A compound of the formula

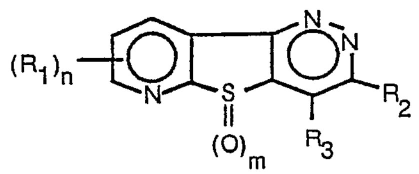

wherein

$R_1$ is alkyl, alkenyl, alkenyl, halogen, nitro, cyano, carboxy, carboxyalkyl, carbalkoxy, alkanoyl, trihalomethyl, aryl, aralkyl, mercapto, mercaptoalkyl, alkylmercapto, hydroxy, hydroxyalkyl, alkoxy, acyloxy, amino, aminoalkyl, alkylamino, sulfinyl, sulfonyl or methylenedioxy,

m is in interger from 0 to 2 inclusive,

n is in interger from 0 to 3 inclusive, and

$R_2$ and $R_3$ are independently hydrogen, alkyl, alkenyl, alkynyl, hydroxy, hydroxyalkyl, alkoxy, acyloxy, halogen, amino, aminoalkyl, alkylamino, alkanoylamino, mercapto, mercaptoalkyl, alkylmercapto, carboxy, carbalkoxy, alkylsulfinyl, alkylsulfonyl, arylsulfinyl, or arylsulfonyl, wherein the alkyl, alkenyl and alkynyl groups contain up to 6 carbon atoms, and the aryl groups contains from 6 to 10 carbon atoms, and salts of said compounds.

2. A compound of the formula

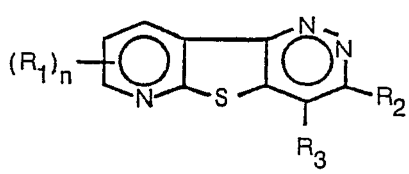

wherein

n is an integer from 0 to 3 inclusive,

$R_1$ is alkyl, alkenyl, alkynyl, halogen, nitro, cyano, carboxy, carboxyalkyl, alkanoyl, trihalomethyl, aryl, aralkyl, mercapto, mercaptoalkyl, alkylmercapto, hydroxy, hydroxyalkyl, alkoxy, acyloxy, amino, aminoalkyl, alkylamino, sulfinyl or sulfonyl, and

$R_2$ and $R_3$ are independently hydrogen, alkyl, alkenyl, alkynyl, hydroxy, hydroxyalkyl, alkoxy, acyloxy, halogen, amino, aminoalkyl, alkylamino, alkanoylamino, mercapto, mercaptoalkyl, alkylmercapto, carboxy, carbalkoxy, alkylsulfinyl, alkylsulfonyl, arylsulfinyl, or arylsulfonyl, wherein the alkyl, alkenyl and alkynyl groups contains up to 6 carbon atoms, and the aryl groups contains from 6 to 10 carbon atoms, and salts of said compounds.

3. A compound of the formula

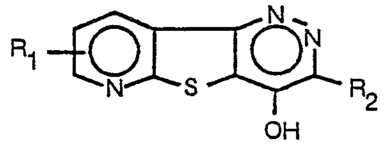

wherein

$R_1$ is alkyl containing 1 to 6 carbon atoms, and

$R_2$ is hydrogen, carboxy or carbalkoxy, and salts of said compounds, especially, acid addition salts.

4. A compound according to any of Claims 1—3 wherein $R_1$ is methyl.

5. A compound according to any of Claims 1—4 wherein $R_2$ is hydrogen.

6. A compound according to any of Claims 1—4 wherein $R_2$ is carboxy.

7. A compound according to any of Claims 1—4 wherein $R_2$ is carbalkoxy.

8. A compound according to any of Claims 1—4 wherein $R_2$ is carbethoxy.

9. 3-Carboxy-4-hydroxy-7-methyl-pyrido[3′,2′:4,5]thieno[3,2—C]pyridazine.

10. An acid addition salt of the compound of Claim 9.

11. The process of preparing compounds of formula I herein which comprises

a. diazotization of a compound of formula II;

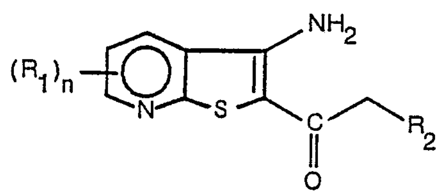

II

b. ring closure of a compound of formula V herein with hydrazine;

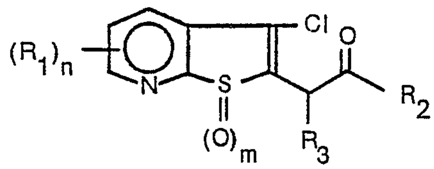

V

c. diazotization and ring closure of a compound of formula VI herein; or

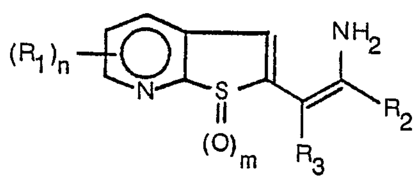

VI

d. by ring closure of a compound of formula VII or VIII herein by elimination of hydrogen halide and aromatization of the resulting product;

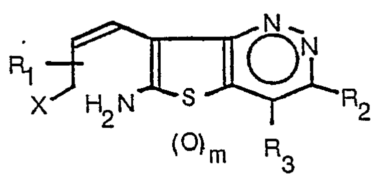

VII

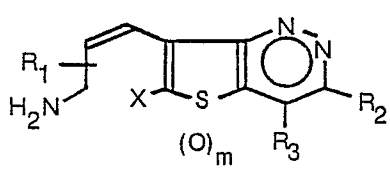

VIII

in which $R_1$, $R_2$, $R_3$, n and m are as defined in claim 1 and X is halogen and optionally forming substituents on the compounds so produced by substitution or conversion reactions;

and optionally forming salts of the said compounds with acids and bases preferably pharmaceutically-acceptable acid addition salts.

12. Process as in Claim 11 wherein the product is a compound of formula I in which m = 0.

13. Process as in Claim 11 wherein the product is a compound of formula I in which n = 1; m = 0—2; $R_1$ is alkyl; $R_2$ is hydrogen, carboxy, or carbalkoxy and $R_3$ = OH.

14. Process as in Claim 11 wherein the product is a compound of formula I in which n = 1; m = 0—2; $R_1$ is methyl; $R_2$ is hydrogen, carboxy, or carbalkoxy and $R_3$ = OH.

15. Process as in any of Claims 11—14 wherein the product is a compound of formula I in which n = 1; m = 0—2; $R_1$ is alkyl; and $R_2$ is carbonyl.

16. Process as in any of Claims 11—14 wherein the product is a compound of formula I in which n = 1; m = 0—2; $R_1$ is alkyl; and $R_2$ is carbalkoxy.

17. Process as in any of Claims 11—14 wherein the product is a compound of formula I in which n = 1; m = 0—2; $R_1$ is alkyl; and $R_2$ is carbethoxy.

18. Process as in any of Claims 11—14 wherein the product is a compound of formula I in which n = 1; m = 0—2; $R_1$ is alkyl; and $R_2$ is hydrogen.

19. Process and in Claim 11 wherein the product is 3-carboxy-4-hydroxy-7-methyl-pyrido[3',2':4,5]thieno[3,2—C]pyridazine.

20. Process as in Claim 11 wherein the product is an acid addition salt of 3-carboxy-4-hydroxy-7-methyl-pyrido[3',2':4,5]thieno[3,2—C]pyridazine.

21. Compounds according to Claims 1 to 10 for use in the treatment of allergies and related conditions.

## Patentansprüche

1. Verbindung der allgemeinen Formel

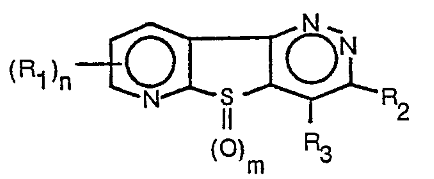

I

worin bedeuten:

$R_1$ Alkyl, Alkenyl, Alkinyl, Halogen, Nitro, Cyano, Carboxy, Carboxyalkyl, Carbalkoxy, Alkanoyl, Trihalogenmethyl, Aryl, Aralkyl, Mercapto, Mercaptoalkyl, Alkylmercapto, Hydroxy, Hydroxyalkyl, Alkoxy, Acyloxy, Amino, Aminoalkyl, Alkylamino, Sulfinyl, Sulfonyl oder Methylendioxy,

m eine ganze Zahl von 0 bis einschließlich 2,

n eine ganze Zahl von 0 bis einschließlich 3, und

$R_2$ und $R_3$ unbabhängig voneinander Wasserstoff, Alkyl, Alkenyl, Alkinyl, Hydroxy, Hydroxyalkyl, Alkoxy, Acyloxy, Halogen, Amino, Aminoalkyl, Alkylamino, Alkanoylamino, Mercapto, Mercaptoalkyl, Alkylmercapto, Carboxy, Carbalkoxy, Alkysulfinyl, Alkylsulfonyl, Arylsulfinyl oder Arylsulfonyl, wobei die Alkyl-, Alkenyl- und Alkinylgruppen bis zu 6 Kohlenstoffatome und die Arylgruppen 6 bis 10 Kohlenstoffatome enthalten; und Salze dieser Verbindungen.

2. Verbindung der allgemeinen Formel

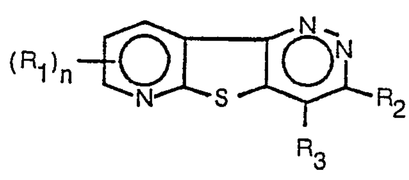

worin bedeuten:

n eine ganze Zahl von 0 bis einschließlich 3,

$R_1$ Alkyl, Alkenyl, Alkinyl, Halogen, Nitro, Cyano, Carboxyl, Carboxyalkyl, Alkanoyl, Trihalogenmethyl, Aryl, Aralkyl, Mercapto, Mercaptoalkyl, Alkylmercapto, Hydroxy, Hydroxyalkyl, Alkoxy, Acyloxy, Amino, Aminoalkyl, Alkylamino, Sulfinyl oder Sulfonyl, und

$R_2$ und $R_3$ unabhängig voneinander Wasserstoff, Alkyl, Alkenyl, Alkinyl, Hydroxy, Hydroxyalkyl, Alkoxy, Acyloxy, Halogen, Amino, Aminoalkyl, Alkylamino, Alkanoylamino, Mercapto, Mercaptoalkyl, Alkylmercapto, Carboxy, Carbalkoxy, Alkylsulfinyl, Alkylsulfonyl, Arylsulfinyl oder Arylsulfonyl, wobei die Alkyl-, Alkenyl- und Alkinylgruppen bis zu 6 Kohlenstoffatome und die Arylgruppen 6 bis 10 Kohlenstoffatome enthalten; und Salze dieser Verbindungen.

3. Verbindung der allgemeinen Formel

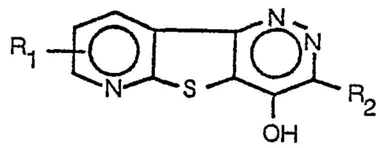

worin bedeuten:

$R_1$ Alkyl mit 1 bis 6 Kohlenstoffatomen,

$R_2$ Wasserstoff, Carboxy oder Carbalkoxy;

und Salze dieser Verbindungen, insbesondere Säure-Additionssalze.

4. Verbindung nach einem der Ansprüche 1 bis 3, dadurch gekennzeichnet, daß $R_1$ Methyl bedeutet.

5. Verbindung nach einem der Ansprüche 1 bis 4, dadurch gekennzeichnet, daß $R_2$ Wasserstoff bedeutet.

6. Verbindung nach einem der Ansprüche 1 bis 4, dadurch gekennzeichnet, daß $R_2$ Carboxy bedeutet.

7. Verbindung nach einem der Ansprüche 1 bis 4, dadurch gekennzeichnet, daß $R_2$ Carbalkoxy bedeutet.

8. Verbindung nach einem der Ansprüche 1 bis 4, dadurch gekennzeichnet, daß $R_2$ Carbethoxy bedeutet.

9. 3-Carboxy-4-hydroxy-7-methyl-pyrido[3',2':4,5]thieno[3,2—C]pyridazin.

10. Säure-Additionssalz der Verbindung gemäß Anspruch 9.

11. Verfahren zur Herstellung von Verbindungen der allgemeinen Formel (I), umfassend

a) die Diazotierung einer Verbindung der nachstehenden Formel (II)

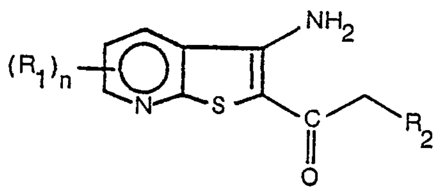

II

b) die Ringschlußbildung einer Verbindung der nachstehenden Formel (V) mit hydrazin

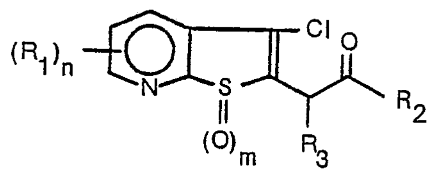

V

c) die Diazotierung und Ringschlußbildung einer Verbindung der nachstehenden Formel (VI)

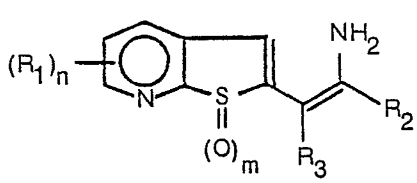

VI

oder

d) die Ringschlußbildung einer Verbindung der nachstehenden Formel (VII) oder (VIII) durch Eliminieren von Halogenwasserstoff und Aromatisierung des resultierenden Produkts

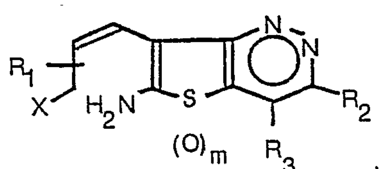

VII

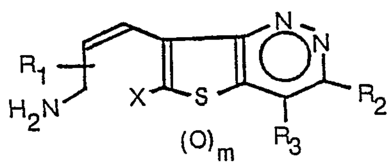

VIII

worin

$R_1$, $R_2$, $R_3$, n und m wie in Anspruch 1 definiert sind und X Halogen bedeutet, sowie wahlweise Anbringen von Substituenten an den so hergestellten Verbindungen durch Substitutions- oder Umwandlungsreaktionen;
und wahlweise Bilden von Salzen dieser Verbindungen mit Säuren und Basen, vorzugsweise pharmazeutisch annehmbare Säure-Additionssalze.

12. Verfahren nach Anspruch 11, dadurch gekennzeichnet, daß das Produkt eine Verbindung der Formel (I) ist, worin m = 0.

13. Verfahren nach Anspruch 11, dadurch gekennzeichnet, daß das Produkt eine Verbindung der Formel (I) ist, worin bedeuten:
n = 1,
m = 0 bis 2,
$R_1$ Alkyl,
$R_2$ Wasserstoff, Carboxy oder Carbalkoxy und
$R_3$ = OH.

14. Verfahren nach Anspruch 11, dadurch gekennzeichnet, daß das Produkt eine Verbindung der Formel (I) ist, worin bedeuten:
n = 1,
m = 0 bis 2,
$R_1$ Methyl,
$R_2$ Wasserstoff, Carboxy oder Carbalkoxy und
$R_3$ = OH.

15. Verfahren nach einem der Ansprüche 11 bis 14, dadurch gekennzeichnet, daß das Produkt eine Verbindung der Formel (I) ist, worin bedeuten:
n = 1,
m = 0 bis 2,
$R_1$ Alkyl und
$R^2$ Carbonyl.

16. Verfahren nach einem der Ansprüche 11 bis 14, dadurch gekennzeichnet, daß das Produkt eine Verbindung der Formel (I) ist, worin bedeuten:
n = 1,
m = 0 bis 2,
$R_1$ Alkyl und
$R_2$ Carbalkoxy.

17. Verfahren nach einem der Ansprüche 11 bis 14, dadurch gekennzeichnet, daß Produkt eine Verbindung der Formel (I) ist, worin bedeuten:
n = 1,
m = 0 bis 2,
$R_1$ Alkyl und
$R_2$ Carbethoxy.

18. Verfahren nach einem der Ansprüche 11 bis 14, dadurch gekennzeichnet, daß das Produkt eine Verbindung der Formel (I) ist, worin bedeuten:
n = 1,
m = 0 bis 2,
$R_1$ Alkyl und
$R_2$ Wasserstoff.

19. Verfahren nach Anspruch 11, dadurch gekennzeichnet, daß das Produkt 3-Carboxy-4-hydroxy-7-methyl-pyrido[3′,2′:4,5]thieno[3,2-c]pyridazin ist.

20. Verfahren nach Anspruch 11, dadurch gekennzeichnet, daß das Produkt ein Säure-Additionssalz von 3-Carboxy-4-hydroxy-7-methyl-pyrido[3′,2′:4,5]thieno[3,2-c]pyridazin ist.

21. Verbindungen gemäß den Ansprüchen 1 bis 10 zur Verwendung in der Behandlung von Allergien und verwandten Zuständen.

**Revendications**

1. Composé de formule:

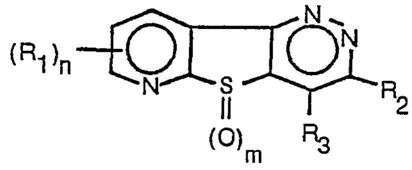

I

dans laquelle:
$R_1$ est un groupe alkyle, alcényle, alcynyle, un halogène, un groupe nitro, cyano, carboxyle,

carboxyalkyle, carbalcoxyle, alcanoyle, trihalogénométhyle, aryle, aralkyle, mercapto, mercaptoalkyle, alkylmercapto, hydroxyle, hydroxyalkyle, alcoxyle, acyloxyle, amine, aminoalkyle, alkylamine, sulfinyle, sulfonyle ou méthylénedioxy,

*m* est un nombre entier de 0 à 2 inclus,

*n* est un nombre entier de 0 à 3 inclus et

$R_2$ et $R_3$ sont, indépendamment, un atome d'hydrogène, un groupe alkyle, alcényle, alcynyle, hydroxyle, hydroxyalkyle, alcoxyle, acyloxyle, un halogène, un groupe amine, aminoalkyle, alkylamine, alcanoylamine, mercapto, mercaptoalkyle, alkylmercapto, carboxyle, carbalcoxyle, alkylsulfinyle, alkylsulfonyle, arylsulfinyle ou arylsulfonyle, dans lesquels les groups alkyle, alcényle et alcynyle contiennent jusqu'à 6 atomes de carbone et les groupes aryle contiennent de 6 à 10 atomes de carbone, ainsi que les sels de ces composés.

2. Composé de formule:

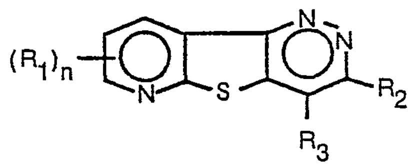

dans laquelle:

*n* est un nombre entier de 0 à 3 inclus,

$R_1$ est un groupe alkyle, alcényle, alcynyle, un halogène, un groupe nitro, cyano, carboxyle, carboxyalkyle, alcanoyle, trihalogénométhyle, aryle, aralkyle, mercapto, mercaptoalkyle, alkylmercapto, hydroxyle, hydroxyalkyle, alcoxyle, acyloxyle, amine, aminoalkyle, alkylamine, sulfinyle ou sulfonyle, et

$R_2$ et $R_3$ sont, indépendamment, un atome d'hydrogène, un groupe alkyle, alcényle, alcynyle, hydroxyle, hydroxyalkyle, alcoxyle, acyloxyle, un halogène, un groupe amine, aminoalkyle, alkylamine, alcanoylamine, mercapto, mercaptoalkyle, alkylmercapto, carboxyle, carbalcoxyle, alkylsulfinyle, alkylsulfonyle, arylsulfinyle ou arylsulfonyle, dans lesquels les groupes alkyle, alcényle et alcynyle contiennent jusqu'à 6 atomes de carbone et les groupes aryle contiennent de 6 à 10 atomes de carbone, ainsi que les sels de ces composés.

3. Composé de formule:

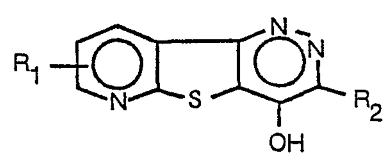

dans laquelle:

$R_1$ est une groupe alkyle contenant 1 à 6 atomes de carbone, et

$R_2$ est un atome d'hydrogène ou un groupe carboxyle ou carbalcoxyle,

ainsi que les sels de ces composés, spécialement les sels d'addition d'acide.

4. Composé selon l'une quelconque des revendications 1 à 3, dans lequel $R_1$ est un groupe méthyle.

5. Composé selon l'une quelconque des revendications 1 à 4, dans lequel $R_2$ est l'hydrogène.

6. Composé selon l'une quelconque des revendications 1 à 4, dans lequel $R_2$ est un groupe carboxyle.

7. Composé selon l'une quelconque des revendications 1 à 4, dans lequel $R_2$ est un groupe carbalcoxyle.

8. Composé selon l'une quelconque des revendications 1 à 4, dans lequel $R_2$ est un groupe carbéthoxyle.

9. La 3-carboxy-4-hydroxy-7-méthyl-pyrido[3',2':4,5]thiéno[3,2—C]pyridazine.

10. Sel d'addition d'acide du composé selon la revendication 9.

11. Procédé de préparation de composés de formule I ici donnée, qui consiste:

a) à diazoter un composé de formule II:

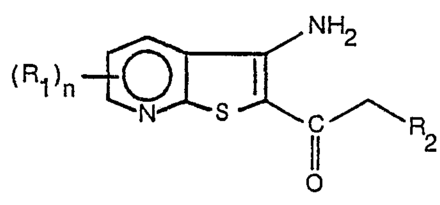

II

b) à cycliser au moyen d'hydrazine un composé de formule V:

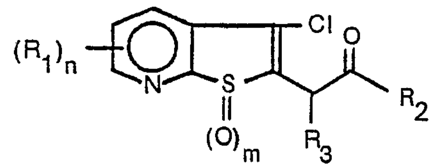

V

c) à diazoter et à cycliser un composé de formule VI:

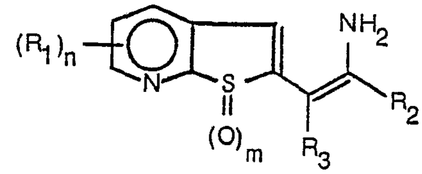

VI

d) ou à cycliser un composé de formule VII ou VIII par élimination d'halogénure d'hydrogène et à aromatiser le produit obtenu:

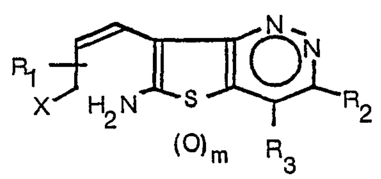

VII

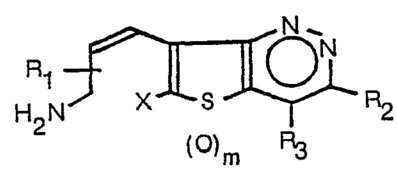

VIII

formules dans lesquelles $R_1$, $R_2$, $R_3$, *n* et *m* sont tels que définis à la revendication 1 et X est un halogène, et, facultativement, à former des substituants sur les composés ainsi préparés, par des réactions de substitution ou de conversion:
et facultativement, à former des sels de ces composés avec des acides et des bases, de préférence des sels d'addition d'acide pharmaceutiquement acceptables.

12. Procédé selon revendication 11, dans lequel le produit est un composé de formule I dans lequel $m = 0$.

13. Procédé selon la revendication 11, dans lequel le produit est un composé de formule I dans lequel $n = 1$; $m = 0$ à 2, $R_1$ est un groupe alkyle, $R_2$ est un atome d'hydrogène ou un groupe carboxyle ou carbalcoxyle et $R_3 = OH$.

14. Procédé selon la revendication 11, dans lequel le produit est une composé de formule I, dans laquelle $n = 1$; $m = 0$ à 2, $R_1$ est un groupe méthyle, $R_2$ est un atome d'hydrogène ou un groupe carboxyle ou carbalcoxyle et $R_3 = OH$.

15. Procédé selon l'une quelconque des revendications 11 à 14, dans lequel le produit est un composé de formule I, dans laquelle $n = 1$; $m = 0$ à 2, $R_1$ est groupe alkyle et $R_2$ est un groupe carbonyle.

16. Procédé selon l'une des revendications 11 à 14, dans lequel le produit est un composé de formule I, dans laquelle $n = 1$; $m = 0$ à 2, $R_1$ est un groupe alkyle et $R_2$ est un groupe carbalcoxyle.

17. Procédé selon l'une des revendications 11 à 14, dans lequel le produit est un composé de formule I, dans laquelle $n = 1$; $m = 0$ à 2, $R_1$ est un groupe alkyle et $R_2$ est un groupe carbéthoxyle.

18. Procédé selon l'une des revendications 11 à 14, dans lequel le produit est un composé de formule I, dans laquelle $n = 1$; $m = 0$ à 2, $R_1$ est un groupe alkyle et $R_2$ est un atome d'hydrogène.

19. Procédé selon la revendication 11, dans lequel le produit est la 3-carboxy-4-hydroxy-7-méthyl-pyrido[3',2':4,5]thiéno[3,2—C]pyridazine.

20. Procédé selon la revendication 11, dans lequel le produit est un sel d'addition d'acide de la 3-carboxy-4-hydroxy-7-méthyl-pyrido[3',2':4,5]thiéno[3,2—C]pyridazine.

21. Composé selon les revendications 1 à 10, pour l'utilisation dans le traitement d'allergies et d'états apparentés.